# EUROPÄISCHE PATENTANMELDUNG

(11) **EP 4 374 896 A1**
(43) Veröffentlichungstag der Anmeldung: **29.05.2024**
(21) Anmeldenummer: 22209712.3
(22) Anmeldetag: 25.11.2022
(51) Int. Cl.: A61M 5/315, A61M 5/32, A61M 5/20

(54) **ANTRIEBSEINHEIT FÜR EINE MEDIZINISCHE SPRITZE, SPRITZE MIT EINER DERARTIGEN ANTRIEBS-EINHEIT UND AUTOINJEKTOR**

(71) Anmelder: Inductio AG, 4500 Solothurn (CH)
(72) Erfinder: KOLLER, Horst, 8730 Uznach (CH)
(74) Vertreter: Tergau & Walkenhorst Intellectual Property GmbH

(57) **Zusammenfassung**

Eine Antriebseinheit (10) für eine einen zur Aufnahme eines medizinischen Wirkstoffs vorgesehenen Spritzenkörper (2, 2', 2") umfassende medizinische Spritze (1) soll in besonders einfach gehaltener Bauweise für den Einsatz mit einer Vielzahl von Spritzenkörpern, insbesondere auch gängigen Spritzenkörpern, geeignet sein. Dazu umfasst die Antriebseinheit (10) erfindungsgemäß eine auf einen im Spritzenkörper (2, 2', 2") geführten Stopfen (18, 18', 18") wirkende, mit einer vorgespannten Sprungfeder (38) versehene Kolbenstange (30), deren Arretierung mittels eines elastisch verformbaren Federelements (50) der Antriebseinheit (10) bewirkt ist.

## Beschreibung

Die Erfindung bezieht sich auf eine Antriebseinheit für eine medizinische Spritze. Sie betrifft weiterhin eine medizinische Spritze mit einer solchen Antriebseinheit sowie einen Autoinjektor.

Mit medizinischen Substanzen oder Wirkstoffen vorbefüllte Behältnisse, wie beispielsweise vorgefüllte Spritzen oder vorgefüllte Karpulen, kommen im medizinischen Bereich in großem Umfang zum Einsatz. Solche Container oder Spritzen werden üblicherweise von den Herstellern in kontrollierten sterilen Umgebungen befüllt, so dass der Verwender, insbesondere der Patient selbst oder das medizinische Fachpersonal, sie vor der Verwendung nicht mehr selbst aus einem Vorrat, beispielsweise aus einer Vorratsflasche oder - ampulle, befüllen muss. Vorgefüllte Spritzen haben in der Regel eine Haltbarkeit von zwei Jahren oder mehr.

Ein Einsatz solcher vorbefüllter Behältnisse oder Spritzen zur Verabreichung von Medikamenten oder Wirkstoffen kann, wie bei sonstigen Spritzen auch, durch medizinisch geschultes Fachpersonal vorgenommen werden. Um dabei aber im Sinne einer zügigen Bearbeitung eine besonders effiziente Bearbeitung durch das Fachpersonal oder aber auch eine Verabreichung durch den Patienten selbst, also ohne das Erfordernis, das Fachpersonal überhaupt hinzuzuziehen, zu ermöglichen, können mit Wirkstoff vorbefüllte Spritzen in Kombination mit einem geeigneten Antriebselement in Form eines so genannten Autoinjektors bereitgestellt werden. Solche Autoinjektoren bieten eine Automatisierung des eigentlichen Injektionsvorgangs, wobei mittels einer geeigneten Antriebseinheit die auf den Stopfen in der Spritzeneinheit einwirkende Kolbenstange betätigt und damit der Stopfen in der Spritze verschoben und der Wirkstoff durch die Spritzennadel ausgebracht wird. Durch eine solche Antriebseinheit kann somit der eigentliche Vorgang des Wirkstoffaustrags automatisiert und beispielsweise an Vorgaben hinsichtlich der Austragsgeschwindigkeit oder dergleichen angepasst werden, ohne dass der Bediener dies beeinflussen könnte oder müsste. Insbesondere wird das Medikament abgegeben, ohne dass der Patient oder das medizinische Fachpersonal die Kolbenstange betätigen muss, so dass dies auch von ungeschulten "Laien", also insbesondere dem Patienten selbst, durchgeführt werden kann. Ein weiterer Vorteil solcher Autoinjektorsysteme ist, dass diese als passive Nadelschutzsysteme ausgelegt sein können, um nach der Injektion einen unbeabsichtigten Nadelstich zu vermeiden.

Als Primärpackung oder Primärbehälter für derartige Autoinjektoren werden üblicherweise vorgefüllte Spritzen, die dem Industriestandard "Staked Needle" entsprechen, wie die BD Hypak, die Gerresheimer RTF oder ClearJect, die Terumo Plajex, die Daikyo Crystal Zenith ^{®} Spritze, und andere handelsübliche Glas- oder füllfertige Spritzen aus Glas oder Kunststoff, verwendet. Der auf die Kolbenstange und über diese auf den im Spritzenkörper geführten Stopfen wirkende Antrieb ist in solchen Systemen üblicherweise als vorgespannte Feder ausgeführt, die sich beim Vortrieb des Kolbens entspannt. Ein Autoinjektor, bei dem eine solchermaßen vorgespannte Feder auf den im Spritzenkörper vorgehaltenen Wirkstoff einwirkt und diesen somit unter Überdruck setzt, ist beispielsweise aus der EP 2 922 588 B1 bekannt. Bei diesem Autoinjektor ist aber die Verwendung einer geeignet angepassten Ampulle oder Wirkstoffkammer erforderlich, die die Verwahrung des Wirkstoffs unter Überdruck überhaupt erlaubt und dazu mit einer geeigneten Abdichtung am distalen Ende des Spritzenkörpers versehen ist.

Der Erfindung liegt nunmehr die Aufgabe zugrunde, eine beispielsweise zum Einsatz in einem solchen Autoinjektor geeignete Antriebseinheit für eine medizinische Spritze anzugeben, die in besonders einfach gehaltener Bauweise für den Einsatz mit einer Vielzahl von Spritzenkörpern, insbesondere auch gängigen Spritzenkörpern, geeignet ist.

Diese Aufgabe wird erfindungsgemäß gelöst mit einer Antriebseinheit, die eine auf einen im Spritzenkörper geführten Stopfen wirkende, mit einer vorgespannten Sprungfeder versehene Kolbenstange umfasst, deren Arretierung mittels eines elastisch verformbaren Federelements der Antriebseinheit bewirkt ist.

Vorteilhafterweise ist das Federelement dabei zwei-komponentig ausgeführt und umfasst einen in der Art eines Zylindermantels ausgeführten Grundkörper aus einem vergleichsweise harten Material und einen daran angeordneten Federkörper aus vergleichsweise weichem, elastisch verformbaren Material.

In weiterer vorteilhafter Ausgestaltung ist die Arretierung der Antriebseinheit mittels einer in axialer Richtung der Kolbenstange verschiebbaren Drückerkappe lösbar, wobei die Drückerkappe zur Sicherung gegen unbeabsichtigtes Auslösen von einer Sicherungskappe umschlossen ist, die für eine Entsicherung oder Entriegelung durch Rotation um ihre Längsachse um einen vorgegebenen Entsicherungswinkel ausgelegt ist.

Gemäß einem Aspekt der Erfindung wird die Antriebseinheit in einer medizinischen Spritze mit einem zur Aufnahme eines medizinischen Wirkstoffs vorgesehenen Spritzenkörper verwendet. In weiterer vorteilhafter und als eigenständig erfinderisch angesehener Ausgestaltung ist im Spritzenkörper der medizinischen Spritze ein zweikomponentig ausgeführter Stopfen geführt. Der Stopfen umfasst dabei besonders bevorzugt einen zentralen Stopfenkörper aus einem harten Kunststoff, vorzugsweise aus Polypropylen (PP), der von einem aus im Vergleich dazu weicheren Kunststoffmaterial, vorzugsweise aus TPE, gebildeten Dichtmantel umgeben ist.

Gemäß einem weiteren Aspekt stellt die Erfindung einen Autoinjektor mit einem zur Aufnahme eines medizinischen Wirkstoffs vorgesehenen, in einem verschiebbaren Außenrohr angeordneten Spritzenkörper bereit, der mit einer Antriebseinheit der vorstehend beschriebenen Art bestückt ist.

Weitere vorteilhafte und/oder als eigenständig erfinderisch angesehene Aspekte der Erfindung sind in den Unteransprüchen angegeben und/oder im nachfolgenden Ausführungsbeispiel eingehender erläutert.

Ein Ausführungsbeispiel der Erfindung wird anhand einer Zeichnung näher erläutert. Darin zeigen:
- FIG. 1: eine medizinische Spritze in seitlicher Ansicht,
- FIG. 2: die Spritze nach FIG. 1 im Längsschnitt,
- FIG. 3: die Spritze gemäß FIG. 1 perspektivisch im Längsschnitt (Fig. 3 a mit aufgesetzter Nadelschutzkappe, FIG. 3 b mit abgenommener Nadelschutzkappe),
- FIG. 4: perspektivisch im Längsschnitt eine Antriebseinheit der Spritze gemäß FIG. 1,
- FIG. 5: eine Distanzhülse der Antriebseinheit gem. FIG. 4 in zwei perspektivischen Ansichten (FIG. 5a, 5b),
- FIG. 6: die Distanzhülse nach FIG. 5 mit eingesetztem Federelement,
- FIG. 7: das Federelement nach FIG. 6 in rückseitiger Ansicht,
- FIG. 8: zwei als Riegelelemente vorgesehene Wippenkörper des Antriebselements gem. FIG. 4,
- FIG. 9: das Ensemble nach FIG. 6 mit eingesetzten Wippenkörpern,
- FIG. 10: das Ensemble nach FIG. 9 mit aufgesetzter Drückerkappe,
- FIG. 11: die Drückerkappe nach FIG. 10 in perspektivischer Ansicht von unten,
- FIG. 12: die Drückerkappe nach FIG. 11 mit aufgesetzter Sicherungskappe,
- FIG. 13: das Ensemble nach FIG. 10 mit aufgesetzter Sicherungskappe im Längsschnitt
- FIG. 14: die medizinische Spritze nach FIG. 1 bei der Auslösung der Antriebseinheit,
- FIG. 15: einen im Spritzenkörper nach FIG. 3 geführten Stopfen im Längsschnitt,
- FIG. 16: den Stopfen nach FIG. 15 in perspektivischer Ansicht,
- FIG. 17: den Stopfen nach FIG. 15 in seitlicher Ansicht,
- FIG. 18: den Stopfen nach FIG. 15 in perspektivischer Ansicht,
- FIG. 19: einen Spritzenkörper mit aufgeschraubtem Nadelkopf im Längsschnitt,
- FIG. 20: einen schraubbaren Nadelkopf der Spritze gem. FIG. 1 in perspektivischer Ansicht,
- FIG. 21: den Nadelhalter gem. FIG. 20 im Längsschnitt (FIG. 21a) bzw. in seitlicher Ansicht (FIG. 21b),
- FIG. 22: den Nadelhalter gem. FIG. 20 mit aufgesteckter Nadelschutzkappe in perspektivischer Ansicht,
- FIG. 23: einen alternativen, als Schnappverschluss ausgestalteten Nadelhalter der Spritze gem. FIG. 1 in seitlicher Ansicht,
- FIG. 24: den Nadelhalter gem. FIG. 23 im Längsschnitt,
- FIG. 25: den Nadelhalter gem. FIG. 23 mit aufgesteckter Nadelschutzkappe im Längsschnitt (FIG. 25a) und in seitlicher Ansicht (FIG. 25b),
- FIG. 26: einen Spritzenkörper mit vorläufig aufgestecktem Ensemble gem. FIG. 25 im Längsschnitt,
- FIG. 27: einen Spritzenkörper mit aufgestecktem Ensemble gem. FIG. 25 im Längsschnitt,
- FIG. 28: den Nadelkopf der Spritze gemäß FIG. 1 in perspektivischer Ansicht,
- FIG. 29: den Nadelkopf der Spritze gemäß FIG. 1 in seitlicher Ansicht,
- FIG. 30: den Nadelkopf der Spritze gemäß FIG. 1 in einem Zwischenzustand in seitlicher Ansicht,
- FIG. 31: den Nadelkopf der Spritze gemäß FIG. 1 in einsatzbereitem Zustand in seitlicher Ansicht,
- FIG. 32: den Nadelkopf der Spritze gem. FIG. 1 in entsorgungsbereitem Zustand,
- FIG. 33: die Antriebseinheit nach FIG. 4 mit zwei Varianten eines Spritzenkörpers jeweils im Längsschnitt,
- FIG. 34: zwei Varianten des Spritzenkörpers mit einem herkömmlichen Betätigungselement jeweils im Längsschnitt,
- FIG. 35: einen alternativen, als Doppelkammersystem ausgeführten Spritzenkörper im Längsschnitt,
- FIG. 36: die Stopfen des Spritzenkörpers nach FIG. 35 in seitlicher Ansicht,
- FIG. 37: einen Autoinjektor,
- FIG. 38: den Autoinjektor nach FIG. 37 nach Entfernen der Nadelschutzkappe im einsatzbereiten Zustand,
- FIG. 39: den Autoinjektor nach FIG. 37 nach Zurückziehen seines Schutzrohr fertig zur Injektion, und
- FIG. 40: den Autoinjektor nach FIG. 37 nach Abgabe des Wirkstoffs im Längsschnitt.

Gleiche Teile sind in allen Figuren mit denselben Bezugszeichen versehen.

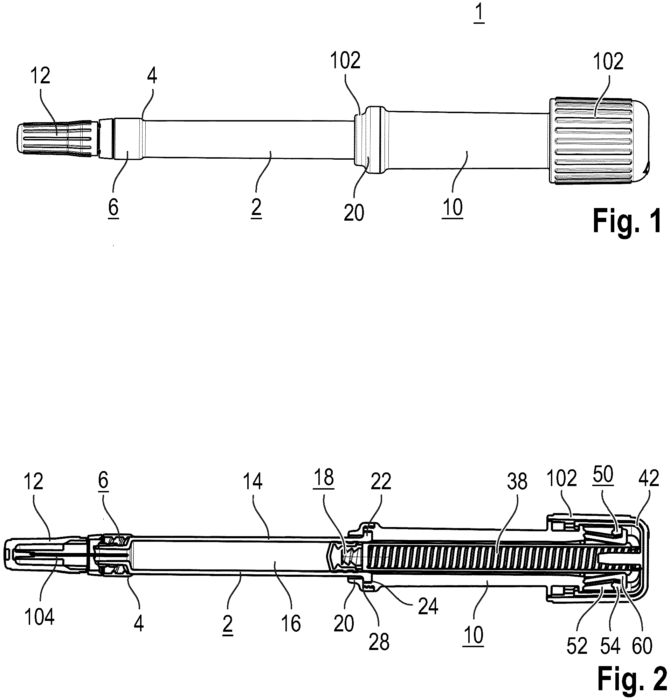

Die medizinische Spritze 1 gemäß FIG. 1 umfasst im Wesentlichen drei Baugruppen, nämlich einen den Spritzenkörper 2 bildenden Wirkstoffbehälter (auch als Kartuschen- oder Patroneneinheit bezeichnet), der einerseits an seinem distalen Ende 4 mit einem Nadelkopf 6 oder Nadelhalter und andererseits an seinem proximalen Ende 8 mit einem Betätigungselement 10 verbunden ist. In der Gesamtansicht in FIG. 1 und auch in der Darstellung im Längsschnitt in FIG. 2 ist die Spritze 1 jeweils mit am Nadelkopf 6 angebrachter Nadelschutzkappe 12 gezeigt. Der Spritzenkörper 2 ist entsprechend einer herkömmlichen Bauweise zylindrisch oder rohrförmig als ein Spritzengehäuse bildender Hohlkörper 14 ausgeführt, der zur Aufnahme des medizinischen Wirkstoffs vorgesehen ist. In diesem Hohlkörper ist innerhalb des die eigentliche Wirkstoffkammer bildenden Spritzenkanals 16 ein Stopfen 18 geführt, der den Innenraum des Hohlkörpers 14 zu seinem proximalen Ende 8 dichtend abschließt. Zudem ist auch der am distalen Ende 4 des Hohlkörpers 14 angebrachte Nadelkopf 6 derart ausgeführt, dass er den Innenraum des Hohlkörpers 14 dichtend abschließt. Der Spritzenkörper 2 ist somit in seiner Gesamtheit als abgedichteter Behälter ausgeführt, in dem der medizinische Wirkstoff für eine gewisse Lagerzeit aufbewahrt und vorgehalten werden kann. Insbesondere kann der Spritzenkörper 2 somit als vorbefüllter Wirkstoffcontainer ausgeführt sein.

Das Betätigungselement 10 ist als automatisiertes Betätigungselement 10 in der Art einer Antriebseinheit 10 für die medizinische Spritze 1 ausgeführt. Gemäß einem als eigenständig erfinderisch angesehenen Aspekt der Erfindung ist die Antriebseinheit 10 dabei als eigenständiges, mit dem Spritzenkörper 2 kombinierbares Bauteil ausgeführt. Mit anderen Worten: gemäß einem Aspekt der Erfindung umfasst die medizinische Spritze 1 einerseits den vorzugsweise vorbefüllten Spritzenkörper 2 und andererseits die mit dem Spritzenkörper 2 verbind- und kombinierbare Antriebseinheit 10. Durch diese Bauweise kann ein herkömmlicher, vorbefüllter, in großer Stückzahl herstellbarer Spritzenkörper 2 durch die Kombination mit der Antriebseinheit 10 für einen automatisierten und damit auch für einen "Laien"-Benutzer, insbesondere den Patienten selbst als Benutzer, durchführbaren automatisierten Austrag des Medikaments oder Wirkstoffs ertüchtigt werden. Ein solches, als eigenständig erfinderisch angesehenes System kann somit als "Auto-Abgabe-Spritze" bezeichnet werden. Im Sinne einer besonders nachhaltigen Bereitstellung solcher automatisierter Systeme kann dabei gemäß einem Aspekt der Erfindung lediglich der Spritzenkörper 2 als Einmal- oder Wegwerfprodukt ausgelegt sein, wohingegen die Antriebseinheit 10 wiederverwendbar im Sinne eines Mehrwegprodukts ausgeführt sein kann. Im Vergleich zu herkömmlichen, einstückig ausgeführten Autoinjektoren kann durch diese Bauweise der entsorgungsbedürftige Anteil der Komponenten deutlich reduziert werden.

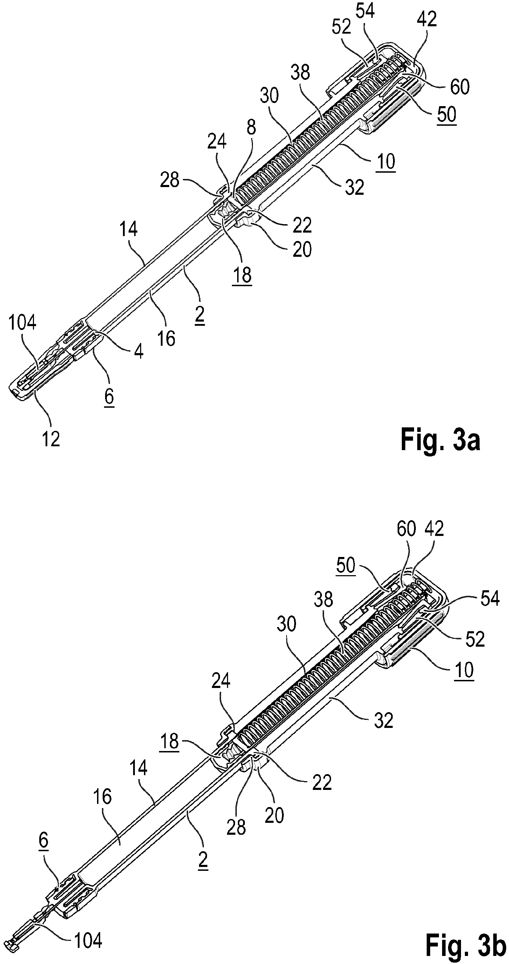

Die Verbindung der Antriebseinheit 10 mit dem Spritzenkörper 2 kann in unterschiedlichen Variationen ausgeführt sein, beispielsweise als Schraub-, Steck- oder Klickverbindung. Im gezeigten Ausführungsbeispiel, insbesondere erkennbar in der Darstellung im perspektivischen Längsschnitt gemäß FIG. 3, ist hierzu in der Art einer Haltekappe ein Überwurfring 20 vorgesehen, der über das distale Ende 4 und den daran angebrachten Nadelkopf 6 auf den Spritzenkörper 2 aufgeschoben werden kann. Am proximalen Ende 8 ist der Spritzenkörper 2 zur Bildung eines Anschlussstutzens mit einem angeformten umlaufenden Kragen 22 versehen, der in eine korrespondierende, endseitig an der Antriebseinheit 10 angeordnete Aufnahmenut 24 eingesteckt werden kann. Der, vorzugsweise mittels eines Gewindes in eine Aufnahmesektion der Antriebseinheit 10 einschraubbare, Überwurfring 20 sitzt dann mit seinem Stirnrand 28 auf dem Kragen 22 auf und fixiert diesen somit in der Aufnahmenut 24, so dass eine feste, aber lösbare Verbindung von Spritzenkörper 2 und Antriebseinheit 10 entsteht.

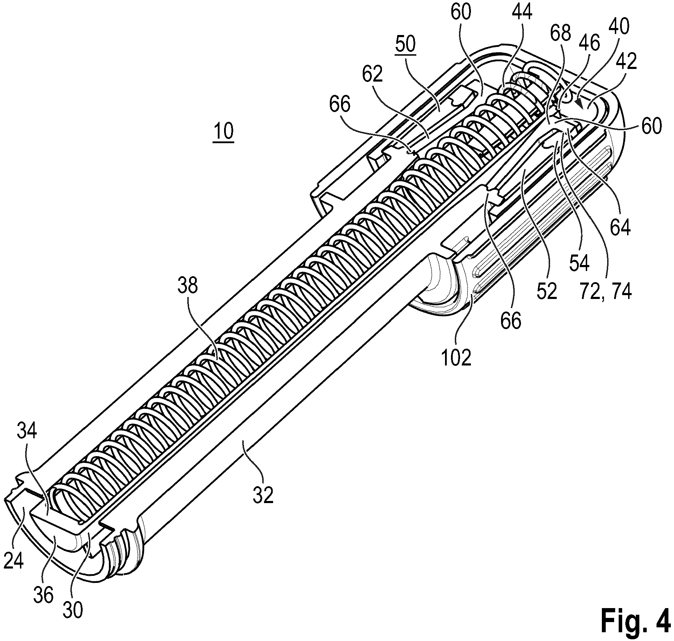

Die als eigenständig erfinderisch angesehene Antriebseinheit 10 ist in FIG. 4 perspektivisch im Längsschnitt gezeigt. Sie umfasst eine als Hohlkörper ausgeführte, in ihrer Längsrichtung verschiebbare Kolbenstange 30. Die Kolbenstange 30 ist in einer ein Au-ßengehäuse der Antriebseinheit 10 bildenden Distanzhülse 32 geführt und in ihrem Außendurchmesser derart an den Spritzenkanal 16 angepasst, dass sie in diesem in Längsrichtung verschiebbar ist. An ihrem distalen Ende 34 weist die Kolbenstange 30 eine Anschlagsfläche 36 auf, die beim Betrieb am Stopfen 18 anliegt und somit auf diesen einwirkt, so dass eine Verschiebung der Kolbenstange 30 in Richtung zum distalen Ende 4 des Spritzenkörpers 2 eine Verschiebung des Stopfens 18 in diese Richtung bewirkt.

Im Inneren der Kolbenstange 30 ist eine im in Fig. 4 gezeigten Zustand unter Vorspannung stehende komprimierte Sprungfeder 38 angeordnet, die sich einerseits an der Rückseite der Anschlagsfläche 36 und andererseits an der inneren Stirnfläche 40 einer Drückerkappe 42 abstützt. Bis zur Auslösung durch den Bediener ist die Kolbenstange 30 einschließlich der darin befindlichen komprimierten Sprungfeder 38 im in Fig. 4 gezeigten Zustand arretiert. Gemäß einem als eigenständig erfinderisch angesehenen Aspekt der Erfindung weist die Kolbenstange 30 an ihrem proximalen, der Drückerkappe 42 zugewandten Ende 44 zu diesem Zweck eine umlaufende Arretierrippe 46 auf.

Als eigenständig erfinderisch angesehen wird die Antriebseinheit 10, bei der die Arretierung und auch das spätere Lösen der Arretierung der Kolbenstange 30 erreicht wird mittels eines elastisch verformbaren Federelements 50. Das Federelement 50 kann gemäß einem Aspekt der Erfindung und wie im Ausführungsbeispiel gezeigt zwei-komponentig ausgeführt sein, wobei es einen in der Art eines Zylindermantels ausgeführten Grundkörper 52 aus einem vergleichsweise harten Material und einen daran angeordneten Federkörper 54 aus vergleichsweise weichem, elastisch verformbaren Material umfasst. Im Ausführungsbeispiel und gemäß einem Aspekt der Erfindung ist der Grundkörper 52 dabei aus PP gebildet, und der Federkörper 54 besteht aus vergleichsweise weichem TPE. Das zweikomponentige Federelement 50 wirkt dabei gemäß einem Aspekt der Erfindung zusammen mit einer Anzahl von in Umfangsrichtung verteilt um die zentralen Kolbenstange 30 angeordneten, als Riegelelement dienenden Wippenkörpern 60.

Jeder Wippenkörper 60, von denen im Ausführungsbeispiel zwei, einander bezüglich der Kolbenstange 30 gegenüberliegend angeordnet, vorgesehen sind, umfasst dabei in als eigenständig erfinderisch angesehener Ausgestaltung zwei winkelig zueinander angeordnete Schenkel 62, 64. Wie der Darstellung gem. FIG. 4 entnehmbar ist, ist der erste, vergleichsweise lang ausgeführte Schenkel 62 dabei derart dimensioniert und positioniert, dass er im gezeigten noch arretierten Ruhezustand des Systems endseitig in eine oberseitig an der Distanzhülse 32 angebrachte Aufnahmenut 66 eingreift. Im Zentralbereich 68 des Wippenkörpers 60, in dem die Schenkel 62, 64 ineinanderlaufen, endet der erste Schenkel 62 in einer Anschlagsfläche 70, auf der Arretierrippe 46 aufliegt. In dieser Position stützt der erste Schenkel 62 somit über die Arretierrippe 46 der Kolbenstange 30 und die Aufnahmenut 66 der Distanzhülse 32 die Kolbenstange 30 gegenüber der Distanzhülse 32 ab, so dass die Kolbenstange 30 gegenüber der Distanzhülse 32 nicht in Richtung zum distalen Ende 34 hin verschoben werden kann. Das System ist somit arretiert, und die Sprungfeder kann sich nicht entspannen und dabei die Kolbenstange 30 verschieben.

Der zweite, vergleichsweise kurze Schenkel 64 ist gemäß einem Aspekt der Erfindung in diesem Zustand nach außen hin, also von der zentralen Kolbenstange 30 weg, ausgerichtet und liegt mit seiner Unterseite 72 auf dem oberen Rand 74 des Federkörpers 54 auf. Zum Lösen der Arretierung ist dabei gemäß einem Aspekt der Erfindung und in als eigenständig erfinderisch angesehener Ausgestaltung vorgesehen, Druck auf den oberen Rand 74 des Federkörpers 54 auszuüben. Aufgrund der gemäß diesem Aspekt der Erfindung vorgesehenen vergleichsweise weichen Materialbeschaffenheit des Federkörpers 54 verformt sich dieser infolge des ausgeübten Drucks, so dass die Unterseite 72 des zweiten Schenkels 64 des Wippenkörpers 60 seine Fixierung verliert. Der Wippenkörper 60 kann somit ausweichen, beispielsweise durch Abkippen nach außen hin. Damit löst sich die Anschlagsfläche 70 von der Arretierrippe 46, und diese verliert ihre Fixierung in Längsrichtung der Kolbenstange 30. Damit kann sich die Sprungfeder 38 nunmehr entspannen und dabei die Kolbenstange 30 und mit dieser den Stopfen 18 in Richtung zum distalen Ende 4 des Spritzenkörpers 2 hin verschieben.

Die zum Lösen der Arretierung vorgesehene Ausübung eines Drucks auf das zweikomponentige Federelement 50 erfolgt dabei mittels der Drückerkappe 42. Gemäß einem Aspekt der Erfindung ist die Drückerkappe 42 dazu an ihrer inneren Stirnfläche 40 mit einer Anzahl von Aktuatorflächen 76 versehen, die bei Betätigung der Drückerkappe 42 gezielt von oben auf den Federkörper 54 lokal einwirken.

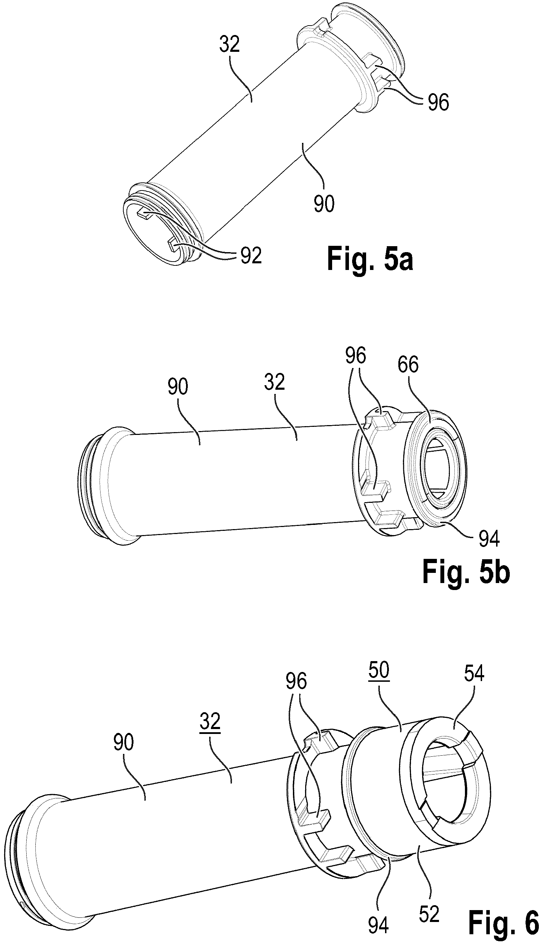

Die erwähnten Komponenten werden nachfolgend anhand der vergrößerten Darstellung in den Figuren noch näher erläutert. So zeigt Fig. 5 in zwei perspektivischen Ansichten die Distanzhülse 32. Diese weist einen in Form eines Zylindermantels ausgestalteten Grundkörper 90 auf, der an seiner Innenseite mit einer Anzahl von Führungsstegen 92 für die Kolbenstange 30 versehen ist (Fig. 5a). In der Darstellung gem. Fig. 5b ist hingegen deutlich die oberseitig angebrachte Aufnahmenut 66 für den ersten Schenkel 62 des jeweiligen Riegelelements 60 erkennbar. Diese ist außenseitig umgeben von einer ringförmig umlaufenden Stützfläche 94 für den Grundkörper 52 des zweikomponentigen Federelements 50. Außenseitig am Grundkörper 90 sind eine Anzahl von Führungsleisten 96 für das Zusammenwirken mit der Drückerkappe 42 angeordnet.

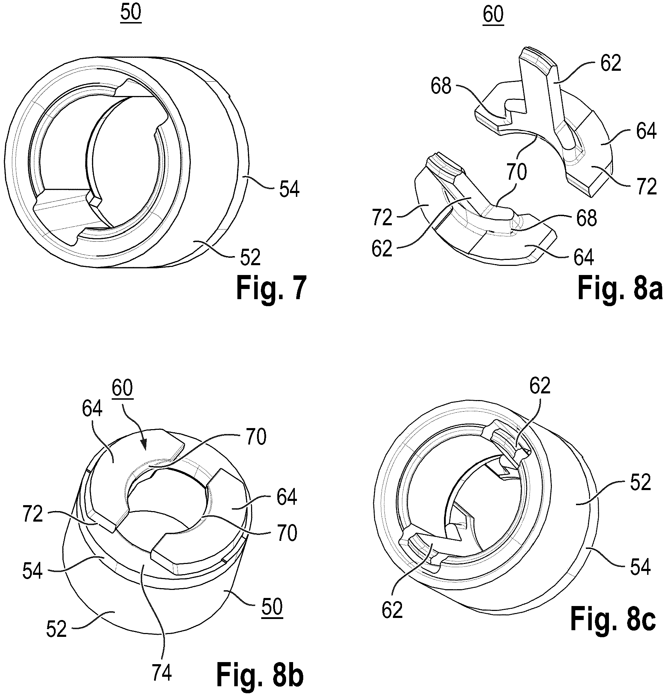

Auf die Stützfläche 94 der Distanzhülse 32 ist das Federelement 50 aufgesetzt, wie dies in der perspektivischen Darstellung nach FIG. 6 erkennbar ist. Der vergleichsweise harte, aus PP gefertigte Grundkörper 52 ist gemäß einem Aspekt der Erfindung in der Art eines Zylindermantels ausgeführt und in seinem Außendurchmesser an den Durchmesser des Grundkörpers 90 der Distanzhülse 32 angepasst, so dass im montierten Zustand lediglich ein geringer Überstand durch die Stützfläche 94 entsteht. In der rückseitigen Ansicht des Federelements 50 gem. FIG. 7 ist gut erkennbar, dass innenseitig in den den Grundkörper 52 bildenden Zylindermantel Aussparungen zur Aufnahme der Wippenkörper 60 eingearbeitet sind.

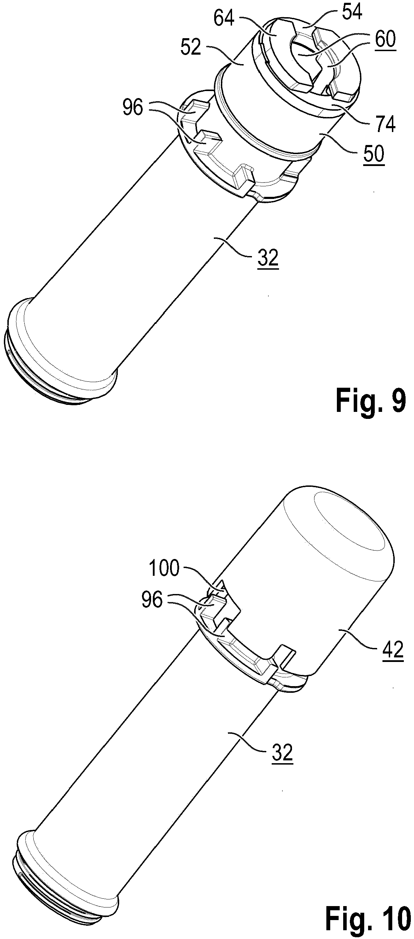

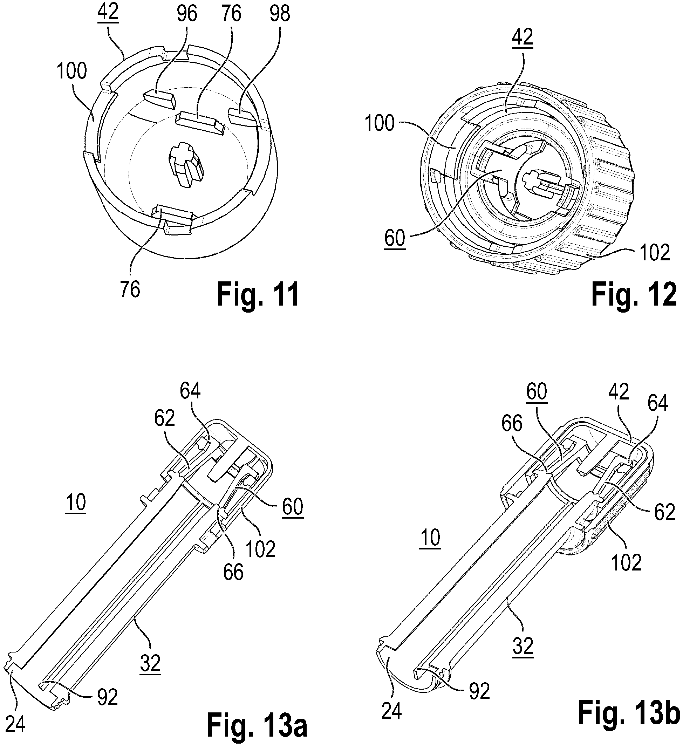

Die Wippenkörper 60, von denen im Ausführungsbeispiel zwei vorgesehen sind, die aber selbstverständlich auch in anderer Anzahl bereitgestellt sein könnten, sind in FIG. 8 in perspektivischer Ansicht (FIG. 8a), in eingesetztem Zustand in perspektivischer Draufsicht (FIG. 8b) und in eingesetztem Zustand in perspektivischer Ansicht von unten (FIG. 8c) gezeigt. FIG. 9 zeigt das Ensemble aus Distanzhülse 32 mit aufgesetztem Federelement 50 gem. FIG. 6 mit nunmehr in das Federelement 50 eingesetzten Wippenkörpern 60. Auf dieses Ensemble wird anschließend die Drückerkappe 42 aufgesetzt, wie dies in FIG. 10 dargestellt ist. Die Drückerkappe 42 ist zur weiteren Verdeutlichung noch einmal in FIG. 11 in perspektivischer Darstellung von unten gezeigt. Deutlich sind dabei die Aktuatorflächen 76 erkennbar, die bei Betätigung der Drückerkappe 42 gezielt von oben auf den Federkörper 54 lokal einwirken sollen. Zudem ist die Drückerkappe 42 an ihrer inneren Stirnfläche 40 mit seitlichen Führungsflächen 98 für die Wippenkörper 60 versehen.

Das in FIG. 10 gezeigte teilmontierte Ensemble ist dafür geeignet, dass durch Drücken der Drückerkappe 42, also durch Belastung der Drückerkappe 42 in Längsrichtung, der Federkörper 54 des Federelements 50 lokal verfomt und die Arretierung der Wippenkörper 60 somit gelöst wird; dieses Ensemble ist somit zum "Auslösen" der Antriebseinheit 10 und ihres durch die Sprungfeder 38 gegebenen Antriebs geeignet. Für eine sichere Führung der Komponenten relativ zueinander sind dabei die außenseitig an der Distanzhülse 32 angebrachten Führungsleisten 96 vorgesehen, die mit korrespondierenden Aussparungen 100 im Mantel der Drückerkappe 42 zusammenwirken. In montiertem Zustand, also mit eingelegter Kolbenstange 30, wäre dieses Ensemble somit auslösebereit. Allerdings ist dieses System nicht gegen ein unbeabsichtigtes und somit fehlerhaftes Auslösen gesichert.

Um dem entgegenzuwirken, umfasst die in FIG. 4 gezeigte Antriebseinheit 10 noch eine Sicherungskappe 102, die das genannte Ensemble außenseitig nahezu vollständig umschließt und gegen eine unbeabsichtigte Auslösung, also eine unbeabsichtigte Verschiebung der Drückerkappe 42 in axialer Richtung, sichert. Gemäß einem als eigenständig erfinderisch angesehenen Aspekt der Erfindung ist die Sicherungskappe 102 dabei für eine Entsicherung oder Entriegelung des Systems durch Rotation, beispielsweise um einen Rotationswinkel von etwa 70°, ausgelegt. Mit anderen Worten: im lagerungsfähigen, gesicherten Zustand ist die Sicherungskappe 102 durch geeignete Führungsmittel, beispielsweise Führungspaarungen von Rippen, Nuten und dergleichen, gegen Verschieben in Längsrichtung blockiert und damit gesichert. Zum Entriegeln dieser Blockierung muss der Nutzer gemäß einem Aspekt der Erfindung die Sicherungskappe 102 um einen vorgegebenen Drehwinkel gegenüber dem restlichen System um die Längsachse verdrehen, beispielsweise bis ein innenseitig angeordneter Anschlag erreicht wird. In dieser verdrehten und entsicherten Position sind die Führungsmittel derart konfiguriert, dass nunmehr ein Verschieben der Sicherungskappe 102 in axialer Richtung und damit ein Auslösen des Systems ermöglicht ist.

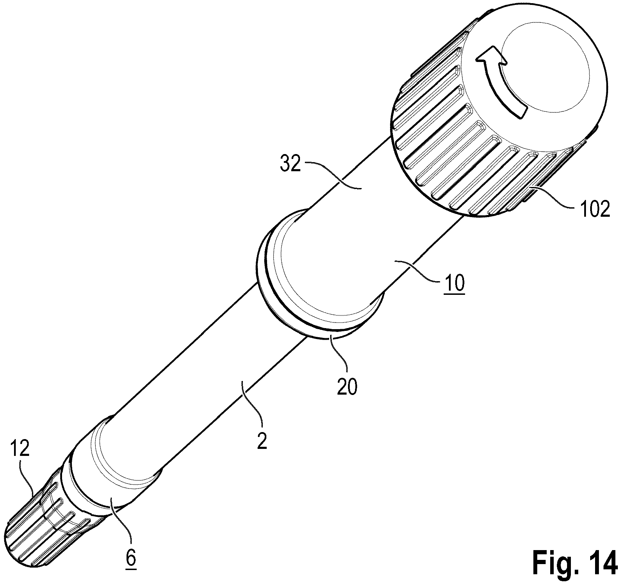

Die mit der Sicherungskappe 102 versehene Drückerkappe 42 ist in FIG. 12 perspektivisch von unten dargestellt; das in FIG. 10 bereits gezeigte Ensemble mit zusätzlich noch aufgebrachter Sicherungskappe 102 ist im Längsschnitt in Fig. 13 gezeigt. Die zur Auslösung der Antriebseinheit 10 vorgesehene Rotation oder Verdrehung der Sicherungskappe 102 um einen Auslösewinkel von etwa 70° ist dagegen in der perspektivischen Darstellung gem. FIG. 14 gezeigt.

Die Antriebseinheit 10 kann, wie bereits in FIG. 3 dargestellt, zur Bildung der medizinischen Spritze 1 geeignet mit einem Spritzenkörper 2, insbesondere einem vorbefüllten Spritzenkörper 2, verbunden werden. In als eigenständig erfinderisch angesehener Ausgestaltung und gemäß einem Aspekt der Erfindung ist dabei der im Spritzenkörper 2 geführte Stopfen 18 zur Erzielung eines synergetischen Effekts an die Antriebseinheit 10 angepasst. Der Stopfen 18 ist dabei über die jeweils am proximalen Ende des Spritzenkörpers 2 angeordnete Antriebseinheit 10 innerhalb des Hohlkörpers 14 verschiebbar, so dass beispielsweise der im Hohlkörper 14 vorgehaltene Wirkstoff über eine im Nadelkopf 6 gelagerte Hohlnadel 104 ausgetragen und somit appliziert werden kann. Der Stopfen 18 ist dabei derart dimensioniert, dass er dichtend an der Innenwand des Hohlkörpers 14 anliegt; im Längsbereich des Innenraums des Hohlkörpers 14 zwischen dem distalen Ende 4 und dem Stopfen 18 wird somit das Reservoir für den Wirkstoff ausgebildet, in dem dieser vorgehalten werden kann. Bei angebrachtem Nadelkopf 6, bei dem die Hohlnadel 104 mit ihrem proximalen Ende in dieses Reservoirvolumen hineinragt, kann der Stopfen 18 innerhalb des Hohlkörpers 14 in Richtung zum distalen Ende 4 hin verschoben werden, so dass der Wirkstoff durch die Hohlnadel 104 aus dem Innenraum herausgedrückt wird.

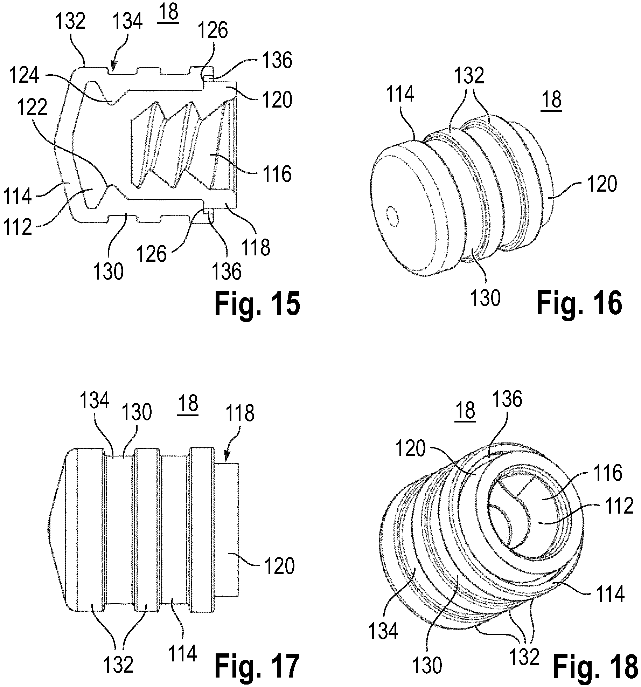

Um auch höchsten Ansprüchen hinsichtlich Dichtheit einerseits und Gleiteigenschaften innerhalb des Hohlkörpers 14, beispielsweise hinsichtlich der Losbrechkraft bei Betätigung des Stopfens 18 gerade in Kombination mit der durch die Sprungfeder 38 der Antriebseinheit 10 aufgebrachten Antriebskraft, andererseits Genüge zu tun, ist der Stopfen 18 in als eigenständig erfinderisch angesehener Ausgestaltung mehrkomponentig, insbesondere zweikomponentig, ausgeführt. Details zur Ausgestaltung des Stopfens 18 sind den Darstellungen im Längsschnitt in FIG. 15, in perspektivischer Ansicht in FIG. 16, in seitlicher Ansicht in FIG. 17 und in weiterer perspektivischer Ansicht "von hinten" in FIG. 18 entnehmbar.

Der gemäß einem Aspekt der Erfindung zweikomponentig oder als "2K-Stopfen" ausgeführte Stopfen 18 umfasst einen zentralen Stopfenkörper 112 aus einem vergleichsweise harten Kunststoff, im Ausführungsbeispiel und gemäß einem Aspekt der Erfindung aus Polypropylen (PP), der von einem aus im Vergleich dazu weicheren Kunststoffmaterial, im Ausführungsbeispiel und gemäß einem Aspekt der Erfindung TPE, gebildeten Dichtmantel 114 umgeben ist. Der zentrale Stopfenkörper 112 dient dabei im Wesentlichen zur Form- und Strukturstabilität und zur Weiterleitung der eingeleiteten Kräfte, insbesondere in Längsrichtung bei der Verschiebung des Stopfens 18 innerhalb des Spritzenkörpers 2. Dazu kann der zentrale Stopfenkörper 112 innenseitig mit einem Aufnahmekanal 116, beispielsweise mit einem Gewinde, versehen sein, über den eine Verbindung mit einem externen Betätigungsmittel, beispielsweise der Antriebseinheit 10 oder einem Betätigungsstößel der Spritze 1, ermöglicht ist. Durch die als eigenständig erfinderisch angesehene Ausführung des Stopfens 18 als 2K-Stopfen mit einer Kombination des zentralen Stopfenkörpers 112 mit dem vergleichsweise weichen Dichtmantel 114 kann mittels geeigneter Geometrie- und Parameterwahl (beispielsweise genaue Materialzusammensetzung oder Auswahl des verwendeten Kunststoffs) dieser Komponenten einerseits die Anpresskraft des Dichtmantels 114 an die Innenwand des Hohlkörpers 14 und somit die Dichtigkeit des Systems ebenso gut und genau eingestellt werden wie die Gleitreibungskräfte beim Verschieben des Stopfens 18 im Hohlkörper. Damit kann ein solchermaßen aufgebauter Stopfen 18 als synergistisches Element in Zusammenwirkung beispielsweise mit der Antriebseinheit 10 im Sinne einer kontrollierten Vorgabe der Systemeigenschaften wie beispielsweise Losbrechkräfte und dergleichen genutzt werden. Des Weiteren kann durch die Kombination dieser Komponenten die üblicherweise bei der Verwendung reiner TPE-Stopfen bei langer Lagerzeit auftretende Schrumpfung und damit ein Dichtigkeitsverlust vermieden werden. Ein solcher 2K-Stopfen ermöglicht somit unter anderem auch zuverlässig vergleichsweise lange Lagerzeiten vorbefüllter Spritzen.

Diese gemäß einem Aspekt der Erfindung vorgesehene Bauweise, also die Bereitstellung des inneren, vergleichsweise härteren Stopfenkörpers 112 mit einer Ummantelung durch den vergleichsweise weicheren Dichtmantel 114, mit dem im Aufnahmekanal 116 vorgesehen stabilen Gewinde wird als besonders vorteilhaft angesehen. Durch diese Bauweise kann der Stopfenkörper 112 und mit diesem der Stopfen 18 insgesamt fest einem Betätigungselement der Spritze 1 verbunden werden. Dadurch können axiale Verschiebungen des Stopfens 18 innerhalb des Hohlkörpers 16 sowohl in "Vorwärts- " als auch in "Rückwärtsrichtung" erfolgen, d. h. es können wahl- und bedarfsweise Schub- oder auch Zugwirkungen auf den Stopfen 18 ausgeübt werden. Damit sind einerseits Anwendungen wie z. B. Aspirationstests und/oder Rekonstitutionen von Medikamenten in einer solchen Spritze ermöglicht. Andererseits können auch vergleichsweise hohe Haltekräfte in einem solchen System toleriert werden, die mit herkömmlichen Stopfenverbindungen nicht möglich wären. Des Weiteren ist üblicherweise beim Einschrauben der Kolbenstange in ein entsprechendes Gewinde eines reinen TPE- oder Gummistopfens ein erhöhter Anpressdruck in Kauf zu nehmen, so dass sich die Haft- oder Gleitreibung entsprechend erhöht. Dieser Nachteil ist durch die gemäß einem Aspekt der Erfindung nunmehr vorgesehene Ausgestaltung des Stopfens 18 nicht mehr in Kauf zu nehmen.

Im vorliegenden Ausführungsbeispiel ist allerdings beim Zusammenwirken mit der Antriebseinheit 10 keine solch innige, in beidseitige Richtung wirkende Verbindung mit dem Stopfen 18 vorgesehen; hier bietet der Grundkörper 112 aufgrund seiner Materialwahl eine zuverlässige Anschlagsfläche für die korrespondierende Anschlagsfläche 36 der Kolbenstange 30.

Der Dichtmantel 114 umgibt den zentralen Stopfenkörper 112 gemäß einem Aspekt der Erfindung in Längsrichtung gesehen nahezu vollständig, wobei lediglich im proximalen Anschlussbereich 118 des Stopfenkörpers 112 ein endseitiger Flansch 120 vom Dichtmantel 114 unbedeckt bleibt. Der zentrale Stopfenkörper 112 ist dabei für eine formschlüssige Fixierung des Dichtmantels 114 mit einer umlaufenden Haltenut 122 versehen, in die eine Innenlippe 124 des Dichtmantels 114 eingreift. Des Weiteren weist der zentrale Stopfenkörper 112 im Bereich des endseitigen Flanschs 120 eine umlaufende Kante 126 auf, auf der eine korrespondierende Innenkante 128 des Dichtmantels 114 aufsitzt.

In einer als eigenständig erfinderisch angesehenen Ausführung ist der Stopfen 18 für eine besonders hohe Dichtwirkung innerhalb des Spritzenkörpers 2 ausgelegt. Dabei wird gemäß einem Aspekt der Erfindung gezielt die relativ gute Verformbarkeit des vergleichsweise weichen Dichtmantels 114 mit einer geeigneten Geometriewahl und Formgebung kombiniert. Dazu weist der Dichtmantel 114 einen im Wesentlichen zylindrischen Außenmantel 130 auf, der mit einer Mehrzahl von in Richtung der Zylinderachse gesehen beabstandet zueinander positionierten umlaufenden Dichtrippen 132 versehen ist. Im Bereich der Dichtrippen 132 kann der Stopfen 18 gemäß einem Aspekt der Erfindung mit einem gewissen Übermaß ausgeführt sein, d. h. einen geringfügig größeren Durchmesser aufweisen als der Innendurchmesser des jeweiligen Spritzenkörpers 2. Aufgrund der Verformbarkeit des den Dichtmantel 114 bildenden Kunststoffs kann dieser sich somit an die Innenkontur des Spritzenkörpers 2 anpassen, wobei aufgrund der Formgebung und der vorgesehenen beabstandet voneinander angeordneten Dichtrippen 132 Ausweichräume bereitgestellt sind, in die die verformten Dichtrippen 132 ausweichen können.

Somit ist es möglich, einerseits auf zuverlässige Weise eine hohe Dichtwirkung zu erzielen, wobei andererseits die auftretenden Haft- und Gleitreibungskräfte infolge des Kontakts mit der Innenwand des jeweiligen Spritzenkörpers 2 gerade im Hinblick auf die mit der Antriebseinheit 10 besonders günstig erreichbaren Antriebskräfte ausreichend geringgehalten werden können. Gemäß einem Aspekt der Erfindung ist dabei die Breite der oder jeder Dichtrippe 132 geringer gewählt als der Abstand zwischen zwei benachbarten Dichtrippen 132. Durch diese Ausgestaltung des Stopfens 18 kann somit bei hoher Dichtigkeit eine besonders gute und störungsfreie Verschiebbarkeit des Stopfens 18 im jeweiligen Spritzengehäuse 2 erreicht werden.

Zusätzlich begünstigt werden diese Effekte durch die gemäß einem Aspekt der Erfindung vorgesehene Positionierung und Ausgestaltung der Haltenut 122 mit korrespondierender Innenlippe 124, die im Schnitt gesehen jeweils eine Art V-förmige Kontur aufweisen. Damit wird unter Nutzung der Verformbarkeit des relativ weichen Materials des Dichtmantels 114 eine Verformungszone 134 geschaffen, in die hinein Material aus der in Vorschubrichtung des Stopfens 18 gesehen vordersten Dichtrippe 132 ausweichen kann. Damit wird der Reibwiderstand des im Spritzenkörper 2 gleitenden Stopfens 18 gemäß einem Aspekt der Erfindung noch weiter verringert.

Zur weiteren Begünstigung des Reibverhaltens des Stopfens 18 weist gemäß einem Aspekt der Erfindung der Dichtmantel 114 im Bereich des endseitigen Flanschs 120 und seiner Innenkante 128 einen im Vergleich zum Außendurchmesser des zentralen Stopfenkörpers 112 geringfügig vergrößerten Innendurchmesser auf, so dass sich im montierten Zustand endseitig ein Spalt in der Art einer umlaufenden Nut 136 zwischen diesen Komponenten ergibt. Durch diese umlaufende Nut 136 wird eine weitere Verformungszone bereitgestellt, in die bei einer Reibbelastung Material der in Vorschubrichtung des Stopfens 18 gesehen letzten Dichtrippe 132 ausweichen kann.

Gemäß einem Aspekt der Erfindung ist der Stopfen 18 durch ein 2K-Spritzgussverfahren hergestellt.

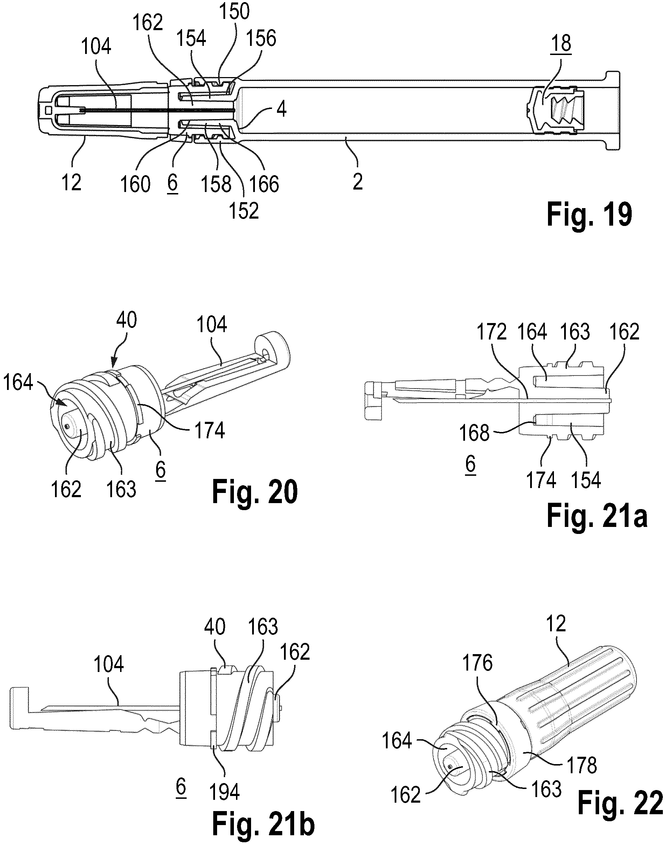

Für die Verbindung des Spritzenkörpers 2 mit dem am distalen Ende 4 des Hohlkörpers 14 angebrachten Nadelkopf 6 sind gemäß Aspekten der Erfindung mehrere Konzepte denkbar, insbesondere eine Schraubverbindung in der Art einer Luer-Verbindung oder eine Schnapp- oder Steckverbindung. Ein Ausführungsbeispiel für den Spritzenkörper 2 mit Schraubverbindung ist in FIG. 19 im Längsschnitt gezeigt. Deutlich ist dabei erkennbar, dass der in dieser Variante an die Konfiguration des Nadelhalters 6 angepasste Spritzenkörper 2 an seinem distalen Ende 4 mit einem Luer-Innengewinde 150 versehen ist. Dieses ist innenseitig an einen endseitigen Anschlussstutzen 152 des Spritzenkörpers 2 angeformt. Der Anschlussstutzen 152 umgibt dabei in seinem Innenbereich ein Verbindungssystem 154 für den Nadelhalter 6. Das Verbindungssystem 154 umfasst gemäß einem Aspekt der Erfindung einen unter Freilassung eines umlaufenden Gewinderaums 156 zentral angeordneten Verbindungszapfen 158, der seinerseits mittig einen sich zum distalen Ende 4 hin aufweitenden Aufnahmekanal 160 für einen zugeordneten, geometrisch an diesen angepassten Steckzapfen 162 des Nadelhalters 6 aufweist. Beim Einschrauben eines korrespondierenden Luer-Außengewindes 163 des Nadelhalters 6 in das daran angepasste Luer-Innengewinde 150 des Spritzenkörpers 2 wird somit der Steckzapfen 162 zunehmend in den Aufnahmekanal 160 eingebracht, bis aufgrund der konischen Ausgestaltung des Steckzapfens 162 einerseits und daran angepasst des Aufnahmekanals 160 andererseits ein flächiger Kontakt zwischen diesen Komponenten entsteht. Damit ist gemäß einem Aspekt der Erfindung eine besonders dichte und formbedingt auch mechanisch besonders stabile Verbindung des Nadelhalters 6 mit dem Spritzenkörper 2 erreicht.

Der Anschlussbereich des Nadelhalters 6 bildet gemäß diesem Aspekt der Erfindung somit eine in Längsrichtung ausgerichtete ringförmige Vertiefung 164 aus, in die der den Aufnahmekanal 160 ringartig umschließende Kragen 166 des Verbindungszapfens 158 eingebracht werden kann. In ihrem Bodenbereich ist diese Vertiefung 164 gemäß einem Aspekt der Erfindung mit einer Anzahl von Rastnocken 168 versehen, in die korrespondierende Nocken an der Stirnfläche des Kragens 166 einschnappen können, sobald das Luer-Gewinde 163 vollständig in das Luer-Innengewinde 150 eingeschraubt wurde. Damit kann die Luer-Verbindung zwischen Spritzenkörper 2 und Nadelhalter 6 nach dem vollständigen Einschrauben rotationsgesichert und somit fixiert werden.

Der Nadelkopf 6 in der ersten, aufschraubbaren Ausführung ist in FIG. 20 in perspektivischer Ansicht und in FIG. 21 im Längsschnitt bzw. in seitlicher Ansicht (FIG. 21b) gezeigt. In dieser Variante ist der Nadelkopf 6 gemäß einem Aspekt der Erfindung in einem Anschlussbereich 40 außenseitig mit dem Luer-Gewinde 163 versehen, das in das angepasste Luer-Innengewinde 150 am distalen Ende 4 des Spritzenkörpers 2 eingeschraubt werden kann. Die zur verbesserten Verbindung mit dem Nadelhalter 6 umspritzt ausgeführte Hohlnadel 104 ist dabei in einem innerhalb des Nadelhalters 6 vorgesehenen Nadellager 172 geführt. Oberhalb des Luer-Gewindes 163 ist der Nadelkopf 6 außenseitig mit einer umlaufenden, segmentweise durchbrochenen Rippe 174 versehen. Auf den Nadelhalter 6 kann gemäß einem Aspekt der Erfindung in der Art einer Vormontage die Nadelschutzkappe 12 aufgesteckt werden, wobei eine Anzahl von innenseitig an der Nadelschutzkappe 12 angeordnete Rastnasen 176 jeweils in eines der ausgesparten Segmente der Rippe 174 eingreifen. Der Nadelhalter 6 und die aufgesteckte Nadelschutzkappe 12 bilden somit ein rotatorisches Gesperre mit Formschluss in Rotationsrichtung, so dass das in FIG. 22 gezeigte Ensemble aus Nadelhalter 6 und aufgesteckter Nadelschutzkappe 12 gemeinsam auf den Spritzenkörper 2 aufgeschraubt werden kann. Dabei wird die Nadelschutzkappe 12 gedreht, und die der mit dem Gewinde 163 versehene Nadelhalter 6 wird durch das Gesperre in Rotationsrichtung mitgenommen, so dass das Gewinde 163 aufgeschraubt wird.

Wie der Darstellung in FIG. 22 zudem entnehmbar ist, ist gemäß einem Aspekt der Erfindung die Nadelschutzkappe 12 als Originalitätsverschluss ausgeführt. Dazu umfasst sie den eigentlichen Kappenkörper, an den in der Art eines Siegels abreißbar ein Sicherungsring 178 angeformt ist. Beim Öffnen der Spritze, also beim Entfernen der Nadelschutzkappe 12 vom Nadelhalter 6, wird der Sicherungsring 178 abgerissen, und er verbleibt auf dem Nadelhalter 6. Damit wird für den Benutzer eindeutig erkennbar, dass die Spritze 1 bereits benutzt und der Wirkstoffcontainer "angebrochen" wurde.

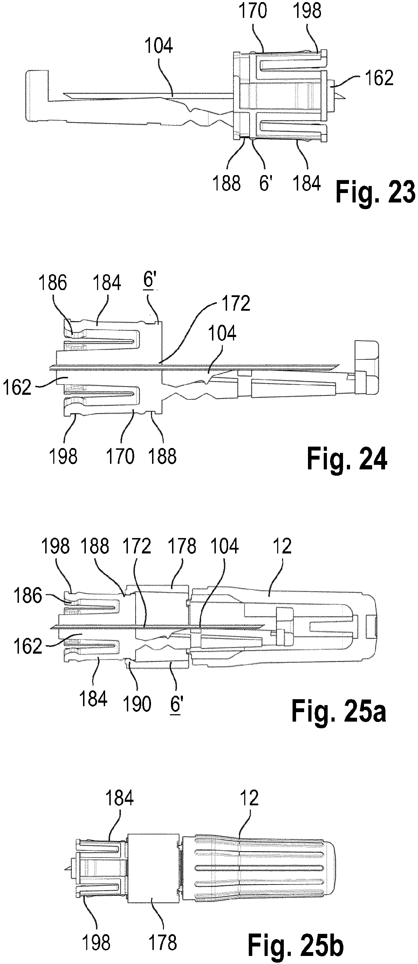

In einer zweiten, als eigenständig erfinderisch angesehenen aufsteckbaren Ausführung ist der Nadelkopf 6' in FIG. 23 in seitlicher Ansicht und in FIG. 24 im Längsschnitt gezeigt. In dieser Variante ist der Nadelkopf 6' gemäß einem Aspekt der Erfindung in seinem Anschlussbereich 170 als Aufprellkappe 184 ausgeführt, an deren Innenumfang eine Anzahl von mit einer zugeordneten Außenwulst im proximalen Endbereich des Spritzenkörpers 2' in Eingriff bringbaren Schnapphaken oder Rastelementen 186 angeordnet ist. Die auch in diesem Ausführungsbeispiel zur verbesserten Verbindung mit dem Nadelhalter 6' umspritzt ausgeführte Hohlnadel 104 ist dabei ebenfalls in einem innerhalb des Nadelhalters 6' vorgesehenen Nadellager 172 geführt. Am oberen Rand der Aufprellkappe 184 ist der Nadelhalter 6' außenseitig mit einer umlaufenden Nut 188 versehen. Auf den Nadelkopf 6' kann gemäß einem Aspekt der Erfindung auch in dieser Variante in der Art einer Vormontage die Nadelschutzkappe 12 aufgesteckt werden, wobei eine Anzahl von innenseitig an der Nadelschutzkappe 12 angeordnete Rastnasen 190 in die Nut 188 eingreifen. Der Nadelhalter 6' und die aufgesteckte Nadelschutzkappe 12 bilden somit das in FIG. 25 im Längsschnitt (FIG. 25a) und in seitlicher Ansicht (FIG. 25b) gezeigte Ensemble, das gemeinsam auf den Spritzenkörper 2' aufgesteckt werden kann.

Wie der Darstellung in FIG. 25 zudem entnehmbar ist, ist gemäß einem Aspekt der Erfindung auch in dieser Variante die Nadelschutzkappe 12 als Originalitätsverschluss ausgeführt. Dazu umfasst sie den eigentlichen Kappenkörper, an den in der Art eines Siegels abreißbar ein Sicherungsring 178 angeformt ist. Beim Öffnen der Spritze, also beim Entfernen der Nadelschutzkappe 12 vom Nadelkopf 6', wird der Sicherungsring 178 abgerissen, und er verbleibt auf dem Nadelhalter 6'. Damit wird für den Benutzer eindeutig erkennbar, dass die Spritze 1 bereits benutzt und der Wirkstoffcontainer "angebrochen" wurde.

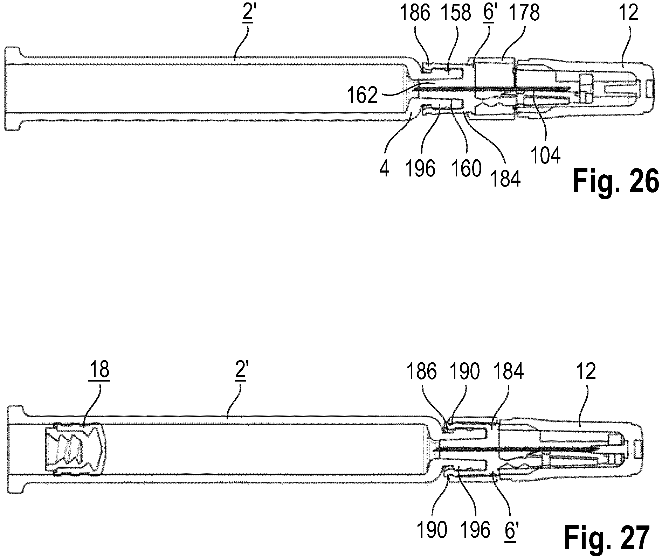

Beim Anbringen der Aufprellkappe 184 kann diese somit auf den mit einer geeignet angepassten Außenwulst 196 versehenen endseitigen Mündungsbereich des Spritzenkörpers 2' aufgeschoben werden, wobei die Rastelemente 186 zunächst durch die Außenwulst 196 nach außen gebogen werden und anschließend, nach weiterem Aufschieben, die Außenwulst 196 hintergreifen und in der Art einer Schnappverbindung mit dieser verrasten. Der solchermaßen mit der teilweise aufgeschobenen Aufprellkappe 184 versehene Spritzenkörper 2' ist in FIG. 26 im Längsschnitt gezeigt. Deutlich ist dabei erkennbar, dass auch in dieser Variante gemäß einem Aspekt der Erfindung der in dieser Variante mit der Außenwulst 196 versehene endseitige Verbindungszapfen 158 des Spritzenkörpers 2' ebenfalls mittig den sich zum distalen Ende 4 hin aufweitenden Aufnahmekanal 160 für den zugeordneten, geometrisch an diesen angepassten, das Nadellager 172 tragenden Steckzapfen 162 des Nadelhalters 6' aufweist. Beim Einstecken des Steckzapfens 162 wird dieser zunehmend in den Aufnahmekanal 160 eingeschoben, bis aufgrund der konischen Ausgestaltung des Steckzapfens 162 einerseits und daran angepasst des Aufnahmekanals 160 andererseits ein flächiger Kontakt zwischen diesen Komponenten entsteht und zudem die Rastelemente 186 mit der Außenwulst 196 verrasten. Dieser Zustand, mit dem gemäß einem Aspekt der Erfindung eine besonders dichte und formbedingt auch mechanisch besonders stabile Verbindung des Nadelkopfs 6' mit dem Spritzenkörper 2' erreicht ist, ist in FIG. 26 gezeigt.

Des Weiteren ist gemäß einem Aspekt der Erfindung der die Aufprellkappe 184 bildende Endbereich des Nadelhalters 6' mit einer im unteren oder Schürzenbereich der Aufprellkappe 184 angeordneten weiteren Nut 198 versehen. Zum endgültigen Verschließen des Spritzenkörpers 2' kann somit, wie dies der Darstellung im Längsschnitt gem. FIG. 27 entnehmbar ist, die Nadelschutzkappe 12 insgesamt und somit auch mit ihrem Sicherungsring 178 noch weiter auf die Aufprellkappe 184 aufgeschoben werden, bis die Rastnasen 190 nunmehr in die Nut 198 eingreifen. Damit ist die Nadelschutzkappe 12 rastend auf dem Nadelkopf 6' fixiert, und der Sicherungsring 178 umschließt den Schürzenbereich der Aufprellkappe 184. Damit wird die rastende Verbindung der die Außenwulst 196 hintergreifenden Rastelemente 186 fixiert, da diese nun nicht mehr nach außen ausweichen können und die Verrastung nunmehr festgelegt ist.

Der an den Spritzenkörper 2, 2' anbringbare Nadelkopf 6, 6', der im Moment der Anbringung noch mit der Nadelschutzkappe 12 versehen ist, weist im Übrigen eine an sich als eigenständig erfinderisch angesehene Ausgestaltung auf. Dabei ist dem Umstand Rechnung getragen, dass es ein zunehmendes Bedürfnis und teilweise auch schon in rechtliche oder regulatorische Vorschriften eingeflossen ist, medizinische Spritzen mit so genannten Nadelschutzsystemen auszurüsten. Zunehmende gesetzliche Vorgaben zur sicheren Arbeitsumgebung in verschiedenen Ländern verpflichten nämlich beispielsweise die Arbeitgeber im medizinischen Bereich zum Schutz ihrer Mitarbeit er vor Nadelstichverletzungen. Dazu kann dann vorgesehen sein, beispielsweise ein System aus Schutzkappen vorzusehen, die über die Nadel gesteckt werden, wenn diese nicht in Benutzung ist, oder es können so genannten Retraktionssysteme vorgesehen sein, bei denen die Nadel nach Gebrauch in das zwischenzeitlich vom Wirkstoff entleerte Spritzengehäuse zurückgezogen wird, so dass die Nadelspitze nicht mehr freiliegt.

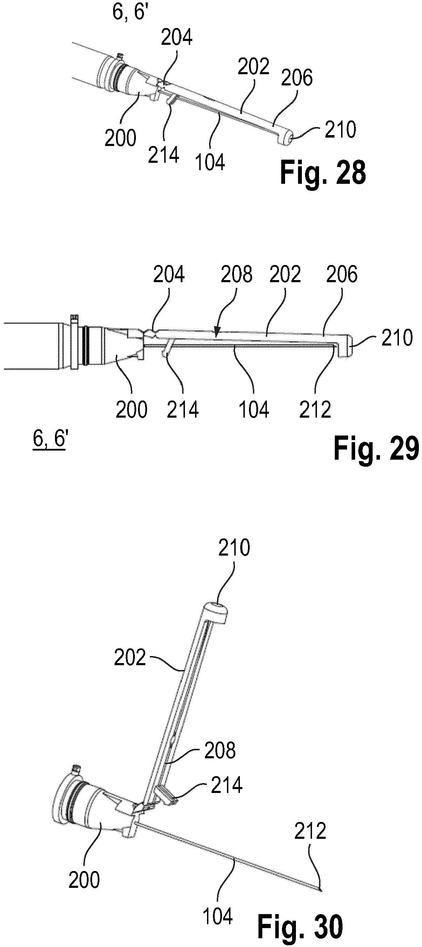

Bei der vorliegenden, als eigenständig erfinderisch angesehenen Ausführungsform ist der an den Hohlkörper 16 anbringbare Nadelkopf 6,6' oder Nadelhalter mit einem integrierten Nadelschutzsystem versehen. Der Nadelkopf 6,6' - nach Entfernung der Nadelschutzkappe 12 - ist in perspektivischer Ansicht in FIG. 28 und in seitlicher Ansicht in FIG. 29 jeweils vergrößert gezeigt.

Wie diesen Darstellungen gut entnehmbar ist, umfasst der Nadelkopf 6, 6' einen den eigentlichen Nadelhalter bildenden Grundkörper 200, vorzugsweise gefertigt aus einem harten Kunststoff wie beispielsweise PP, POM oder PC. Der Grundkörper 200 lagert und trägt dabei die Hohlnadel 104.

Zur Bereitstellung des gewünschten Nadelschutzes ist am Grundkörper 200 eine Nadelschutzhülle 202 über ein Gelenk 204 schwenkbar angebracht. Die Nadelschutzhülle 202 wird dabei von einem länglich ausgedehnten Schutzkörper 206 gebildet, der in seiner Länge die freie Länge der Hohlnadel 104 übertrifft und diese somit endseitig überragt. Des Weiteren ist die Breite des Schutzkörpers 206 derart gewählt, dass er die Hohlnadel 104 beidseitig nach außen hin deutlich überragt. Der Schutzkörper 206 kann zudem innenseitig eine Aufnahmerinne 208 aufweisen, in die die Hohlnadel 104 in der in den FIG. 28, 29 gezeigten Position des Schutzkörpers 206 eingebettet sein kann. Endseitig ist am Schutzkörper 206 eine Deckplatte 210 angeformt, die in der dort gezeigten Position das freie Ende 212 der Hohlnadel 104 abdeckt. In der in den FIG. 28, 29 gezeigten Position ist das freie Ende 212 der Hohlnadel 104 somit vollständig abgeschirmt, und kein Kontakt und somit auch keine Verletzung durch die Hohlnadel 104 ist damit möglich.

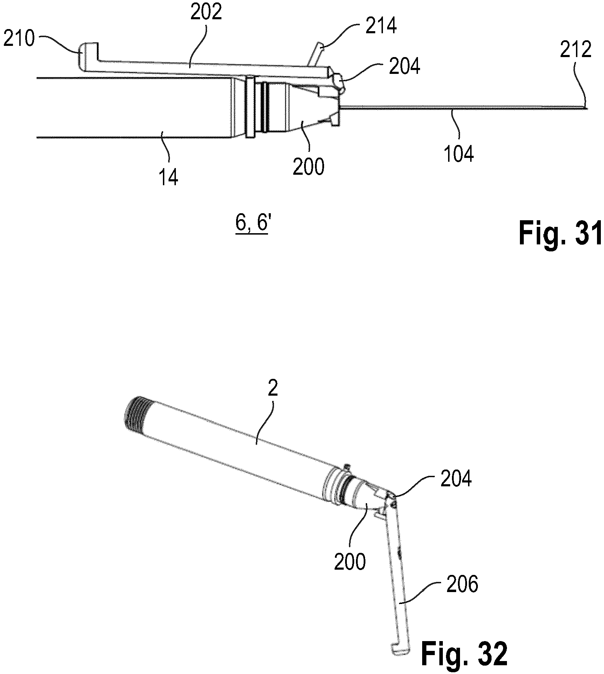

In dieser Position befindet sich das System, wenn die äußere Schutzkappe 12 abgenommen wird. Da die Hohlnadel 104 in diesem Zustand noch von dem Schutzkörper 206 abgeschirmt wird, ist noch kein Einstechen der Nadel 104 möglich. Um diese einsetzbar zu machen, wird daher mittels eines am Schutzkörper 206 angeformten Betätigungselements 214 um das Gelenk 204 herum von der Hohlnadel 104 weggeschwenkt, so dass diese nunmehr freigelegt und freigegeben wird. Zur Verdeutlichung zeigen FIG. 30 eine Zwischenposition mit halb abgeschwenktem Schutzkörper 206 und FIG. 31 eine Endposition, in der der Schutzkörper 206 vollständig von der Nadel 104 abgeschwenkt ist und rückseitig an der Außenseite des Hohlkörpers 14 anliegt. In diesem Zustand ist die Spritze 1 nun zur Abgabe des Wirkstoffs bereit.

Nach Gebrauch der Spritze 1 kann der Schutzkörper 206 um das Gelenk 204 herum wieder zurück geschwenkt werden, so dass er die Hohlnadel 104 erneut wie vorstehend beschrieben abschirmt und damit Verletzungen durch Berührungen der Nadel 104 sicher vermieden sind. Um aber die Verletzungsgefahr an der dann benutzten Nadel 104 noch weiter zu vermindern, ist gemäß einem weiteren Aspekt der Erfindung vorgesehen, den Schutzkörper 206 um das Gelenk 204 herum noch weiter zu verschwenken, bis die Nadel 104 in ihrem Nadellager im Grundkörper 200 abbricht. Damit wird, wie in FIG. 32 dargestellt, die Nadel 104 nunmehr endgültig in ihrer Position innerhalb des Abschirmbereichs des Schutzkörpers 206 fixiert, und zudem wird damit unmittelbar deutlich, dass die Spritze 1 bereits benutzt war und der (nunmehr verbrauchte) Spritzenkörper 2 gemeinsam mit der noch daran montierten, nunmehr zerbrochenen Nadel 104 entsorgt werden soll. Damit sind Irrtümer und Verwechslungen, die beispielsweise zu einem versehentlichen Wiederbenutzen der Spritze 1 führen könnten, sicher ausgeschlossen.

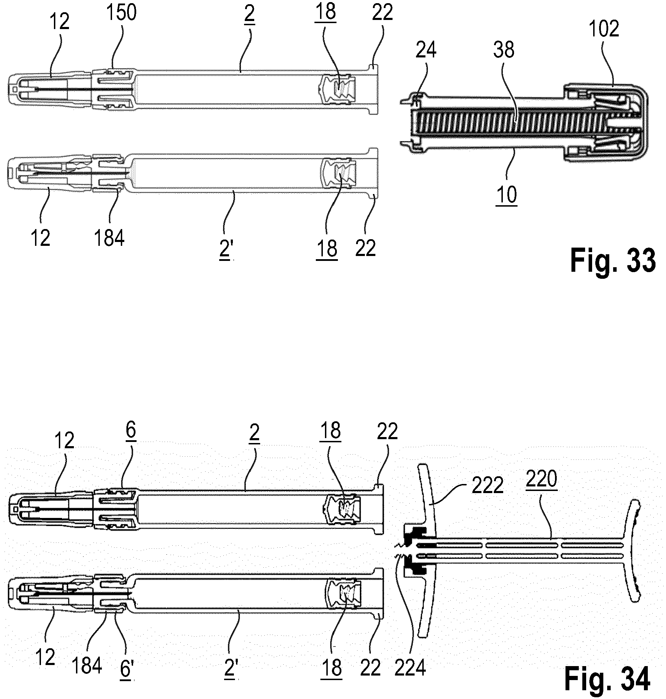

Die Antriebseinheit 10 kann nach einem Aspekt der Erfindung beliebig mit einem Spritzenkörper 2 mit Luer-Verbindung, mit einem Spritzenkörper 2' mit Aufsteck- oder "Snap"-Verbindung der beschriebenen Art oder mit alternativen Ausführungsformen für Spritzenkörper mit alternativer Anbringung des Nadelhalters 6 zur Bildung der medizinischen Spritze 1 kombiniert werden. Gemäß diesem als eigenständig erfinderisch angesehenen Konzept wird somit ein Plattform-System bereitgestellt, bei dem verschiedenartige Typen von Nadelhaltern, mit oder ohne die beschriebene Sicherheitsausführung zum Nadelschutz, jeweils mit Luer-, Steck ("Snap") oder anderweitiger Verbindung zum Spritzenkörper, mit dem Spritzenkörper 2, 2' und der Antriebseinheit 10 kombiniert werden können, je nach Erfordernissen beispielsweise beim Einsatz. Diese Kombinierbarkeit ist schematisch in FIG. 33 durch die gemeinsame Darstellung der beiden Spritzenkörper 2, 2' mit der Antriebseinheit 10 repräsentiert. Das gemäß einem Aspekt der Erfindung vorgesehene Plattformkonzept geht aber noch weiter, denn wie in der analogen Darstellung gem. FIG. 34 gezeigt, können die Spritzenkörper 2, 2' jeweils alternativ auch mit einem manuellen Betätigungselement 220 kombiniert werden. Dieses umfasst in der Art einer herkömmlichen Ausführung einen an einem Griffstück 222 angeordneten Betätigungsstößel 224, der nach der Befestigung des jeweiligen Spritzenkörpers 2, 2' am Griffstück auf den Stopfen 18 wirkt und dessen Verschiebung zum distalen Ende hin ermöglicht.

Das gemäß diesem als eigenständig erfinderisch angesehenen Aspekt bereitgestellte Plattformsystem umfasst daher unterschiedliche Typen von Nadelhaltern 6, 6', Spritzenkörpern 2, 2' und Betätigungselementen 220/Antriebseinheiten 10, die alle geeignet miteinander zur Bildung einer bedarfsgerecht konfigurierten medizinischen Spritze 1 kombiniert werden können. Dies erlaubt unter anderem die Herstellung vorbefüllter Spritzenkörper 2, 2' in einheitlichem Format in enormen Stückzahlen, die erst unmittelbar vor dem Einsatz an entsprechende Vorgaben oder Erfordernisse angepasst und durch Kombination mit dem jeweils geeigneten Nadelhalter 6, 6' und Antrieb 10, 220 konfiguriert werden müssen. Hieraus können sich enorme Effizienz- und Kostenvorteile ergeben.

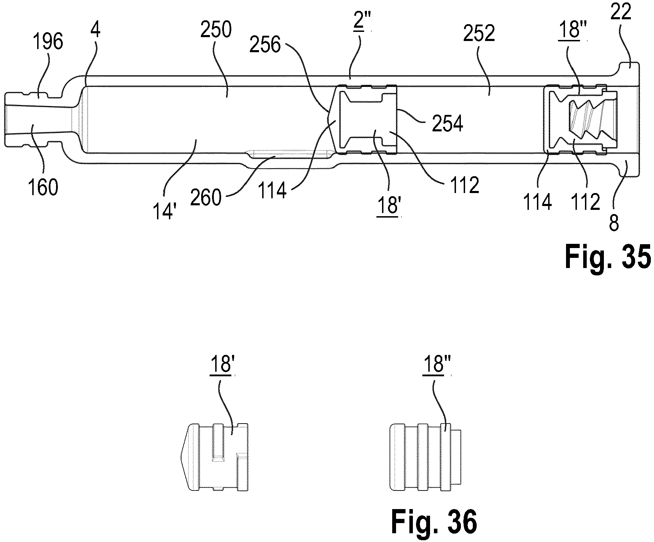

Eine weitere, ebenfalls als eigenständig erfinderisch angesehene Ausführungsform eines Spritzenkörpers 2" ist in FIG. 35 im Längsschnitt gezeigt. Auch in dieser Ausführungsform weist der Spritzenkörper 2" die gleiche äußere Grundform und an seinem proximalen und distalen Ende 4, 8 die passenden Verbindungsmittel auf wie die anderen Ausführungsformen der Spritzenkörper 2, 2'. Auch der Spritzenkörper 2" kann somit problemlos Teil des vorstehend beschriebenen Plattformsystems sein und beliebig mit den Antriebskomponenten und/oder Varianten der Nadelköpfe 6, 6' kombiniert werden. Der Spritzenkörper 2" ist aber als Doppelkammersystem ausgeführt und bildet innerhalb seines Hohlraums 14' zwei durch einen Stopfen 18' getrennte Wirkstoffkammern 250, 252 auf. Ein solches System kann gemäß einem Aspekt der Erfindung in an sich gängiger Weise zur Verabreichung von Wirkstoffen verwendet werden, die unmittelbar vor der Abgabe durch Vermischung zweier Ausgangssubstanzen bereitgestellt werden, wie dies beispielsweise für lyophilisierte Medikamente gängig ist. Der die beiden Wirkstoffkammern 250, 252 trennende Stopfen 18' ist dabei gemäß einem Aspekt der Erfindung ebenfalls, wie vorstehend beschrieben, als 2K-Stopfen ausgestaltet und umfasst einen zentralen, aus einem vergleichsweise harten Material wie PP gebildeten Stopfenkörper 112, der von einem aus einem vergleichsweise weichen Material wie TPE gefertigten Dichtmantel 114 umgeben ist. Funktional bedingt ist in dieser Ausführungsform der Stopfenkörper 112 des Stopfens 18' nicht mit einem inneren Gewinde versehen, sondern weist eine plane Endfläche 254 auf. In Richtung auf das distale Ende 4 des Spritzenkörpers 2" hin bildet der Stopfen 18' ein in einer Spitze 256 mündende Kontur aus, die für die Druckbeaufschlagung des in der Wirkstoffkammer 250 vorgehaltenen Mediums besonders günstig ist.

Am proximalen Ende 8 ist der Hohlraum 14' und damit die Wirkstoffkammer 252 durch den Stopfen 18" verschlossen, der ebenfalls als 2K-Stopfen gemäß der vorstehend beschriebenen Bauweise ausgeführt ist. Dieser Stopfen 18" weist an seiner Vorderseite gemäß einem Aspekt der Erfindung eine plane Fläche 258 auf.

Zur vorgesehenen Vermischung der in den Wirkstoffkammern 250, 252 bereitgestellten Substanzen zur Erzeugung des eigentlichen Wirkstoffs umfasst der Hohlkörper 14' des Spritzenkörpers 2" gemäß einem Aspekt der Erfindung eine innenwandige Ausbuchtung 260. Bei einer Verschiebung des Stopfens 18' innerhalb des Hohlkörpers 14' zu dessen distalem Ende 4 hin gelangt der Stopfen 18' in den Bereich dieser Ausbuchtung 260, so dass er den Innenquerschnitt des Hohlkörpers 14' in diesem Bereich nicht mehr vollständig verschließt. Die Ausbuchtung 260 bildet somit gemäß einem Aspekt der Erfindung einen Überströmkanal aus, über den Medium von der Wirkstoffkammer 252 in die Wirkstoffkammer 250 fließen kann; die Vermischung der Substanzen wird somit ermöglicht.

Die Kombination der Stopfen 18' und 18" ist in FIG. 36 noch einmal in seitlicher Ansicht gezeigt.

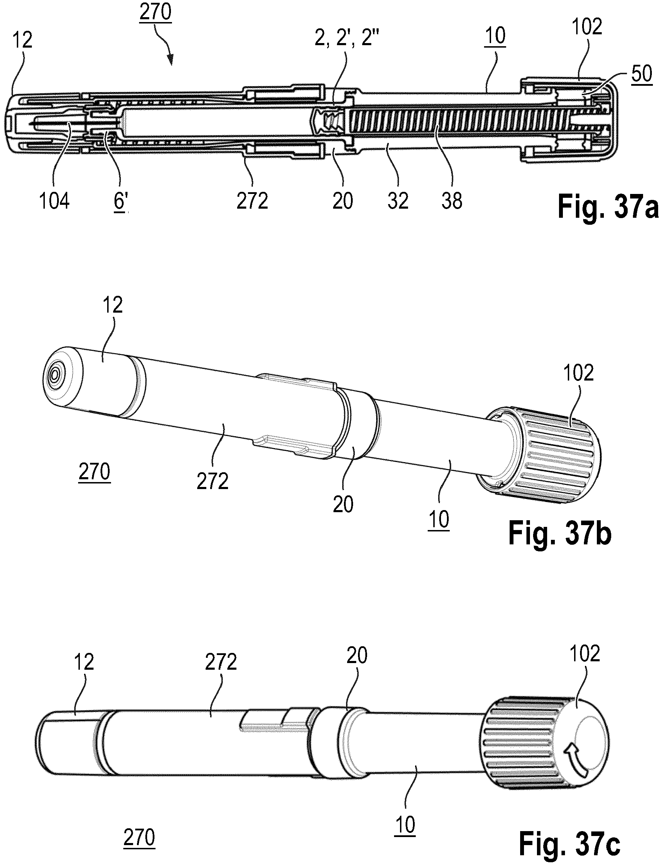

Die vorstehend beschriebene Kombination der Antriebseinheit 10 mit dem jeweiligen Spritzenkörper 2, 2', 2" erlaubt auf einfache Weise die Bereitstellung einer "Auto-Abgabe"-Spritze, bei der unter Rückgriff auf eigentlich standardisierbare Komponenten wie den vorbefüllten Spritzenkörper 2, 2', 2" ein automatisierter Spritzenantrieb zur Verfügung gestellt werden kann, den auch ein "Laien"-Benutzer, insbesondere der Patient selbst, verwenden kann. Dies kann gemäß einem als eigenständig erfinderisch angesehenen Aspekt noch weiter geführt werden, indem auf Basis der beschriebenen Antriebseinheit 10 ein so genannter Autoinjektor bereitgestellt wird. Ein solcher Autoinjektor 270, wie er in FIG. 37 im Längsschnitt (FIG. 37a) und in zwei perspektivischen Ansichten (FIGs. 37b,c) gezeigt ist, ist in seinen wesentlichen Komponenten wie beispielsweise dem Konzept der Antriebseinheit 10, der Ausgestaltung des Spritzenkörpers 2,2', 2" und/oder den Aspekten des Nadelhalters 6, 6' weitgehend baugleich zu der vorstehend beschriebenen, mit der Antriebseinheit 10 versehenen medizinischen Spritze 1. Er umfasst allerdings im Bereich um den Spritzenkörper 2, 2', 2" herum ein mit dem Überwurfring 20 der Antriebseinheit 10 verbundenes Außenrohr 272, das bis zum Bereich des apikalen Endes 4 des Spritzenkörpers 2, 2', 2" geführt und dort mit der Nadelschutzkappe 12 verbunden ist. Dadurch wird somit eine vollständige Kapselung von Nadelkopf 6, 6', Spritzenkörper 2, 2', 2" und Antriebseinheit 10 bereitgestellt, was je nach Einsatzfall eine erhöhte Bedienungssicherheit bieten kann. Allerdings ist bei einer solchen Ausführung die "Vor-Ort"-Konfiguration der Spritze durch Zusammenfügen der genannten Komponenten nicht vorgesehen; vielmehr wird ein solcher Autoinjektor 270 als einstückiges System für den Verwender bereitgestellt.

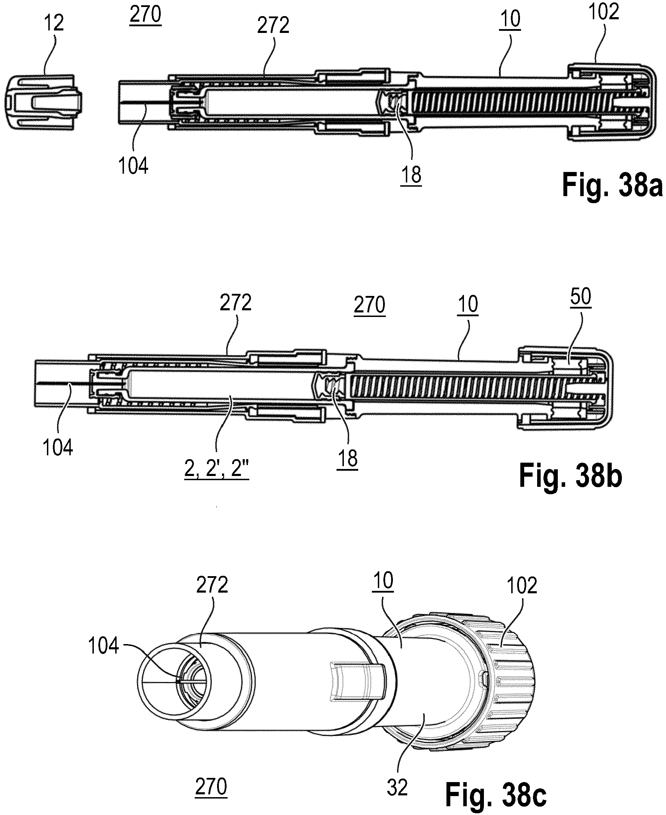

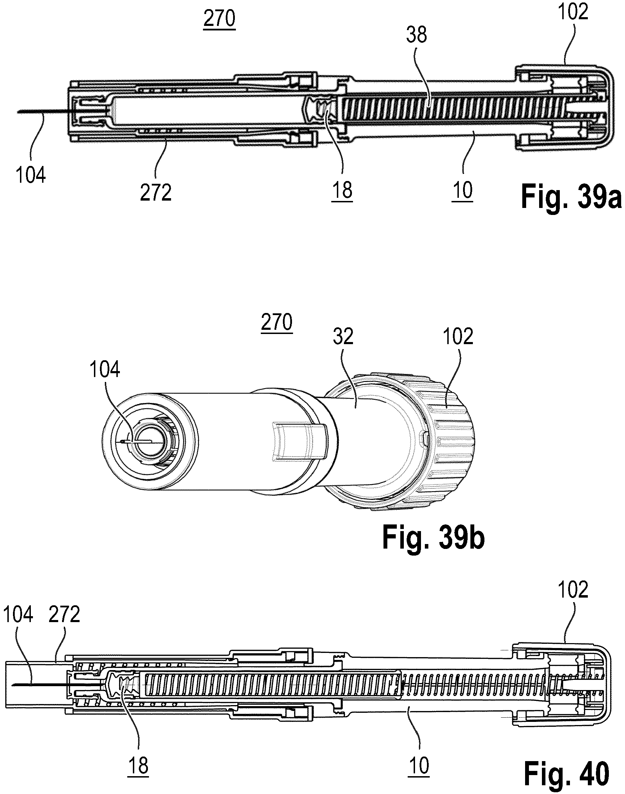

Wie in FIG. 38 dargestellt, wird der Autoinjektor 270 durch Entfernen der Nadelschutzkappe 12 einsatzbereit gemacht. FIG. 38a zeigt dabei das System aus Autoinjektor 270 und Nadelschutzkappe 12 in Explosionsdarstellung im Längsschnitt. FIG. 38b hingegen zeigt den Autoinjektor 270 nach Entfernen der Nadelschutzkappe 12 im Längsschnitt, FIG. 38c in perspektivischer Ansicht. Anschließend wird das als Nadelschutzrohr dienende Außenrohr 272 zurückgezogen, so dass die Hohlnadel 104 freigelegt und der Autoinjektor 270 zur Injektion bereit ist (FIG. 39). FIG. 40 zeigt den Autoinjektor 270 dann nach Verabreichung des Medikaments. Das Außenrohr 272 ist dabei wieder über die Hohlnadel 104 gezogen, so dass diese geschützt und ein unbeabsichtigtes Stechen sicher vermieden ist.

### Bezugszeichenliste

- 1: Medizinische Spritze
- 2: Spritzenkörper
- 4: distales Ende
- 6: Nadelkopf
- 8: proximales Ende
- 10: Betätigungselement
- 12: Nadelschutzkappe
- 14: Hohlkörper
- 16: Spritzenkanal
- 18: Stopfen
- 20: Überwurfring
- 22: Kragen
- 24: Aufnahmenut
- 28: Stirnrand
- 30: Kolbenstange
- 32: Distanzhülse
- 34: distales Ende
- 36: Anschlagsfläche
- 38: Sprungfeder
- 40: innere Stirnfläche
- 42: Drückerkappe
- 44: proximales Ende
- 46: Arretierrippe
- 50: Federelement
- 52: Grundkörper
- 54: Federkörper
- 60: Wippenkörper
- 62, 64: Schenkel
- 66: Aufnahmenut
- 68: Zentralbereich
- 70: Anschlagsfläche
- 72: Unterseite
- 74: oberer Rand
- 76: Aktuatorfläche
- 90: Grundkörper
- 92: Führungssteg
- 94: Stützfläche
- 96: Führungsleiste
- 98: Führungsfläche
- 100: Aussparung
- 102: Sicherungskappe
- 104: Hohlnadel
- 112: Stopfenkörper
- 114: Dichtmantel
- 116: Aufnahmekanal
- 118: Anschlussbereich
- 120: Flansch
- 122: Haltenut
- 124: Innenlippe
- 126: Kante
- 128: Innenkante
- 130: Außenmantel
- 132: Dichtrippe
- 134: Verformungszone
- 136: Nut
- 150: Luer-Innengewinde
- 152: Anschlussstutzen
- 154: Verbindungssystem
- 156: Gewinderaum
- 158: Verbindungszapfen
- 160: Aufnahmekanal
- 162: Steckzapfen
- 163: Luer-Außengewinde
- 164: Vertiefung
- 166: Kragen
- 168: Rastnocken
- 170: Anschlussbereich
- 172: Nadellager
- 174: Rippe
- 176: Rastnase
- 178: Sicherungsring
- 184: Aufprellkappe
- 186: Rastelement
- 188: Nut
- 190: Rastnase
- 196: Außenwulst
- 198: Nut
- 200: Grundkörper
- 202: Nadelschutzhülle
- 204: Gelenk
- 206: Schutzkörper
- 208: Aufnahmerinne
- 210: Deckplatte
- 212: freies Ende
- 214: Betätigungselement
- 220: Betätigungselement
- 222: Griffstück
- 224: Betätigungsstößel
- 250,252: Wirkstoffkammer
- 254: Endfläche
- 256: Spitze
- 258: Fläche
- 260: Ausbuchtung
- 270: Autoinjektor
- 272: Außenrohr

## Patentansprüche

1. Antriebseinheit (10) für eine einen zur Aufnahme eines medizinischen Wirkstoffs vorgesehenen Spritzenkörper (2, 2', 2") umfassende medizinische Spritze (1), die eine auf einen im Spritzenkörper (2, 2', 2") geführten Stopfen (18, 18', 18") wirkende, mit einer vorgespannten Sprungfeder (38) versehene Kolbenstange (30) umfasst, deren Arretierung mittels eines elastisch verformbaren Federelements (50) der Antriebseinheit (10) bewirkt ist.

2. Antriebseinheit (10) nach Anspruch 1, deren Federelement (50) zwei-komponentig ausgeführt ist und einen in der Art eines Zylindermantels ausgeführten Grundkörper (52) aus einem vergleichsweise harten Material und einen daran angeordneten Federkörper (54) aus vergleichsweise weichem, elastisch verformbaren Material umfasst.

3. Antriebseinheit (10) nach Anspruch 1 oder 2, deren Arretierung mittels einer in axialer Richtung der Kolbenstange (30) verschiebbaren Drückerkappe (42) lösbar ist, wobei die Drückerkappe (42) zur Sicherung gegen unbeabsichtigtes Auslösen von einer Sicherungskappe (102) umschlossen ist, wobei die Sicherungskappe (102) dabei für eine Entsicherung oder Entriegelung durch Rotation um ihre Längsachse um einen vorgegebenen Entsicherungswinkel ausgelegt ist.

4. Medizinische Spritze (1) mit einem zur Aufnahme eines medizinischen Wirkstoffs vorgesehenen Spritzenkörper (2, 2', 2"), der antriebsseitig mit einer Antriebseinheit (10) nach einem der Ansprüche 1 bis 3 verbunden ist.

5. Medizinische Spritze (1) nach Anspruch 4, in deren Spritzenkörper (2, 2', 2") ein zweikomponentig ausgeführter Stopfen (18, 18', 18") geführt ist.

6. Medizinische Spritze (1) nach Anspruch 5, deren Stopfen (18, 18', 18") einen zentralen Stopfenkörper (112) aus einem vergleichsweise harten Kunststoff, vorzugsweise aus Polypropylen (PP), umfasst, der von einem aus im Vergleich dazu weicheren Kunststoffmaterial, vorzugsweise aus TPE, gebildeten Dichtmantel (114) umgeben ist.

7. Autoinjektor (270) mit einem zur Aufnahme eines medizinischen Wirkstoffs vorgesehenen, in einem verschiebbaren Außenrohr (272) angeordneten Spritzenkörper (2, 2', 2"), und mit einer Antriebseinheit (10) nach einem der Ansprüche 1 bis 3.
